# EUROPEAN PATENT APPLICATION

(11) **EP 4 692 112 A2**
(43) Date of publication of application: **11.02.2026**
(21) Application number: 25175916.3
(22) Date of filing: 13.05.2025
(51) Int. Cl.: C07K 14/39, C12N 9/24, C12N 9/80, C12N 9/90, C12P 21/02, C12R 1/84

(54) **RECOMBINANT ENGINEERING YEAST FOR PREPARING RECOMBINANT ALBUMIN AND USE THEREOF**

(30) Priority: 07.08.2024 CN 202411075002; 09.12.2024 CN 202411794210
(71) Applicant: Tonghua Anrate Biopharmaceutical Co., Ltd., Tonghua, Jilin 134100 (CN)
(72) Inventor: XIANG, Wei, Tonghua, 134100 (CN); GONG, Guoli, Tonghua, 134100 (CN); HAN, Xu, Tonghua, 134100 (CN); LI, Xiufeng, Tonghua, 134100 (CN); JIANG, Junru, Tonghua, 134100 (CN); GUO, Mingqian, Tonghua, 134100 (CN); GUAN, Tingchen, Tonghua, 134100 (CN)
(74) Representative: Huang, Liwei

(57) **Abstract**

The present invention provides a recombinant engineering bacterium for preparing a recombinant human albumin and use thereof, relates to the field of biochemistry, and particularly relates to the fields of genetic engineering technologies and microorganisms. The present invention comprises any one of the following operations while expressing a recombinant albumin: (1) conducting targeted knockout on one or more genes of SCW10 and UTH1 by utilizing a CRISPR-Cas9 gene editing technology, and/or overexpressing and integrating one or more genes of BGL2 and RCE3 into a strain; and (2) simultaneously overexpressing and integrating an SUS1 gene and an SUA7 gene into a strain, and/or conducting targeted knockout on one or more genes of YHP1 and HDA1 by utilizing the CRISPR-Cas9 gene editing technology. The recombinant engineering bacterium of the present invention significantly improves the yield of the recombinant human albumin, and has wide application prospects.

## Description

### TECHNICAL FIELD

The present invention relates to the field of biochemistry, particularly to the fields of genetic engineering technologies and microorganisms, and more particularly to a recombinant engineering bacterium for preparing a recombinant albumin and use thereof.

### BACKGROUND

As a model eukaryote, the yeast is simple in genetic manipulation, relatively perfect in protein post-translation modification and secretion pathways, rapid in growth speed and low in nutrition requirements, and is suitable for large-scale high-density fermentation. Studies on secretion expression of a large amount of different heterologous proteins in the yeast have proved that the expression levels of different proteins vary greatly. Some heterologous proteins have a limited secretion ability, thereby limiting the production efficiency of proteins. Improvement of a key gene for biosynthesis and secretion mechanisms of a protein through gene manipulation is a major means for improving the production efficiency of the heterologous protein.

Transcription is the first stage for gene expression, and is also a main stage for gene regulation. Through the transcription process, cells transform information of genes into RNA, and then proteins are synthesized through a translation process. Such process is critical for normal functions and development of cells. In the process of intranuclear transcription, transcription factors play a key regulatory role, which enhance or reduce the transcription levels of target genes by binding specific sequences, and function as activators or suppressors. At present, there have been reports about the improvement of the yield of engineering bacteria by regulating transcription factors, for example, in CN111087459A, the yield of artemisinin is improved through overexpression of teosinte branched 1/cycloidea/proliferating cell factor (TCP) family transcription factors; while in CN116836822A, the yield of an echinocandin compound is improved by introducing a transcription factor McfJ into a host cell.

A cell wall is an important component of a yeast cell, which is a barrier for isolating cells from the outside world, is capable of maintaining cell morphology, and plays a crucial role in cell growth, division, conditioned stress and cell fusion mating and maintenance of cell integrity, etc. Many studies have found that changes in components and a structure of the cell wall affect the secretion of the protein. A cell wall protein gene CWP2 disrupting strain is constructed by using recombinant saccharomyces cerevisiae for producing heterologous β-glucosidase as a starting strain. After fermentation for 96 h, the enzyme activity of extracellular β-glucosidase is improved by 53%, and the intracellular enzyme activity is improved by 208% (Li Jie, Disrupting Cell Wall Protein CWP2 Gene to Improve Extracellular Enzyme Activity of Recombinant Saccharomyces Cerevisiae β-glucosidase [J]. Microbiological Bulletin, 2020, 47 (03): 681-690). Through the knockout of a relevant protein DFG5 of a saccharomyces cerevisiae cell wall, SHENZHEN INSTITUTES OF ADVANCED TECHNOLOGY CHINESE ACADENY OF SCIENCES (CN113493800B) improves the secretion expression activity of heterologous protein β-glucosidase by 3 times. Through the knockout of a synthesis relevant gene KIGAS1-1 of a kluyveromyces lactis cell wall, China Agricultural University has found that the thickness of the cell wall is reduced, and the secretion amount of exogenous protein xylanase B is improved (Lian Zhao, Study on the Modification and Protein Secretion Mechanism of Heterozygous Xylanase B Engineering Bacteria Expressed by Kluyveromyces Lactis Yeast [D]. China Agricultural University, 2015).

The above cases provide good examples for constructing recombinant engineering bacteria highly expressing recombinant albumins.

### SUMMARY

The present invention provides a recombinant engineering bacterium for preparing a recombinant albumin. The recombinant engineering bacterium further comprises any one of the following operations while expressing the recombinant albumin:
A1, conducting targeted knockout on one or more genes of SCW10 and UTH1 by utilizing a CRISPR-Cas9 gene editing technology, and/or overexpressing and integrating one or more genes of BGL2 and RCE3 into a strain; and
A2, simultaneously overexpressing and integrating an SUS1 gene and an SUA7 gene into a strain, and/or conducting targeted knockout on one or more genes of YHP1 and HDA1 by utilizing the CRISPR-Cas9 gene editing technology.

In the present invention, the efficient expression of the recombinant albumin is achieved by modifying the cell wall or gene transcription factor of the recombinant engineering bacterium, thereby laying the foundation for the industrial application of the recombinant albumin.

Further, the operation in A1 is to modify the cell wall of the yeast cell to obtain a recombinant engineering bacterium with cell wall deficiency, thereby promoting the expression of the albumin; and the operation in A2 is to modify a transcription factor, wherein the transcription factor is divided into a positive regulatory factor and a negative regulatory factor according to acting results. SUS1 and SUA7 are positive regulatory factors, also called transcription activating factors, which affect the assembling and transcription efficiency of a transcription starting complex and determine whether a gene is expressed, and play a crucial role in transcription regulation of eukaryotic genes. Therefore, the transcription level of the target gene is enhanced by overexpressing SUS1 and SUA7. YHP1 and HDA1 are negative regulatory factors, also called transcription suppressors, which prevent gene transcription. Therefore, gene transcription is promoted through knockout of YHP1 and HDA1, thereby improving the expression of exogenous proteins.

Further, the amino acid sequence encoded by each gene is as follows: the amino acid sequence encoded by the SCW10 gene is as shown in SEQ ID NO.2; the amino acid sequence encoded by the UTH1 gene is as shown in SEQ ID NO.4; the amino acid sequence encoded by the BGL2 gene is as shown in SEQ ID NO.6; the amino acid sequence encoded by the RCE3 gene is as shown in SEQ ID NO.8; the amino acid sequence encoded by the SUS1 gene is as shown in SEQ ID NO.10; the amino acid sequence encoded by the SUA7 gene is as shown in SEQ ID NO.12; the amino acid sequence encoded by the YHP1 gene is as shown in SEQ ID NO.14; and the amino acid sequence encoded by the HDA1 gene is as shown in SEQ ID NO.16.

Further, the nucleotide sequence of each gene is a nucleotide sequence encoding the amino acid sequence of each gene, including nucleotide sequences that have been publicly available or nucleotide sequences that are optimized according to actual needs. Optionally, the nucleotide sequence of the SCW10 gene is as shown in SEQ ID NO.1; the nucleotide sequence of the UTH1 gene is as shown in SEQ ID NO.3; the nucleotide sequence of the BGL2 gene is as shown in SEQ ID NO.5; the nucleotide sequence of the RCE3 gene is as shown in SEQ ID NO.7; the nucleotide sequence of the SUS1 gene is as shown in SEQ ID NO.9; the nucleotide sequence of the SUA7 gene is as shown in SEQ ID NO.11; the nucleotide sequence of the YHP1 gene is as shown in SEQ ID NO.13; and the nucleotide sequence of the HDA1 gene is as shown in SEQ ID NO.15.

In specific embodiments of the present invention, the SCW10-gRNA sequence is as shown in SEQ ID NO.23; the UTH1-gRNA sequence is as shown in SEQ ID NO.24; the YHP1-gRNA sequence is as shown in SEQ ID NO.25; and the HDA1-gRNA sequence is as shown in SEQ ID NO.26.

Further, the recombinant engineering bacterium at least comprises 1 copy of nucleotide sequence encoding the recombinant albumin, optionally, the copy number of the nucleotide sequence encoding the recombinant albumin in the recombinant engineering bacterium obtained in the operation A1 is 3-5. In the embodiment of the present invention, the copy number of the nucleotide sequence encoding the recombinant human albumin in the recombinant engineering bacterium is 3.

Further, the recombinant engineering bacterium also comprises a drug resistance gene fragment and/or a signal peptide sequence. Optionally, the drug resistance gene fragment is at least one selected from a geneticin G418 resistance gene and a Blasticidin resistance gene. Optionally, the signal peptide sequence is a saccharomyces cerevisiae a mating factor signal peptide.

Further, in the recombinant engineering bacterium obtained in the operation A1, a recombinant expression cassette encoding one or more genes of BGL2 and RCE3 is located on a nucleic acid construct with the same selective marker or a nucleic acid construct with different selective markers. In an embodiment of the present invention, the recombinant expression cassette encoding one or more genes of BGL2 and RCE3 is located on the same nucleic acid construct.

Further, in the recombinant engineering bacterium obtained in the operation A2, a recombinant expression cassette encoding SUS1 and SUA7 genes is located on a nucleic acid construct with the same selective marker. In an embodiment of the present invention, the recombinant expression cassette encoding SUS1 and SUA7 genes is located on the same nucleic acid construct.

Further, the recombinant engineering bacterium conducts targeted knockout on relevant genes by utilizing the CRISPR-Cas9 gene editing technology, which comprises the following steps:
S1: constructing a Cas9 and gRNA co-expression plasmid;
S2: preparing donor DNA;
S3: preparing a competent cell; and
S4: constructing any one of the following strains:
   S4-1: knocking out one or more genes of SCW10 and UTH1; and
   S4-2: knocking out one or more genes of YHP1 and HDA1.

Further, the nucleic acid construct comprises any one selected from pHIL-D2, pPIC3.5, pHIL-S1, pPIC9, pPink-LC, pPink-HC, pGAPZA and pGAPZaA plasmids. A recombinant promoter is preferably a constitutive promoter, including any one or more of a GAP promoter, a TEF1 promoter, a PGK1 promoter and an HGT1 promoter.

Further, the recombinant engineering bacterium is one or more selected from Pichia pastoris, Hansenula polymorpha, Candida parapsilosis and Saccharomyces cerevisiae. Optionally, the recombinant engineering bacterium is Pichia pastoris.

Further, the recombinant albumin expressed by the recombinant engineering bacterium obtained in the operation A1 is a recombinant human albumin; and the albumin expressed by the recombinant engineering bacterium obtained in the operation A2 is at least one selected from a recombinant human albumin, a recombinant cat albumin, a recombinant canine albumin, a recombinant bovine albumin and a recombinant horse albumin.

The national center for biotechnology information (NCBI) accession number of the recombinant human albumin is NP_000468.1, and the nucleotide sequence encoding the amino acid sequence of the recombinant human albumin is as shown in SEQ ID NO.17 or SEQ ID NO.18, or has at least 98% identity to the nucleotide sequence as shown in SEQ ID NO.17 or SEQ ID NO.18.

The NCBI accession number of the recombinant cat albumin is NP_001009961.1, and the nucleotide sequence encoding the amino acid sequence of the recombinant cat albumin is as shown in SEQ ID NO.19, or has at least 98% identity to the nucleotide sequence as shown in SEQ ID NO.19;
the NCBI accession number of the recombinant canine albumin is NP_001003026.1, and the nucleotide sequence encoding the amino acid sequence of the recombinant canine albumin is as shown in SEQ ID NO.20, or has at least 98% identity to the nucleotide sequence as shown in SEQ ID NO.20;
the NCBI accession number of the recombinant bovine albumin is NP_851335.1, the nucleotide sequence encoding the amino acid sequence of the recombinant bovine albumin is as shown in SEQ ID NO.21, or has at least 98% identity to the nucleotide sequence as shown in SEQ ID NO.21; and
the NCBI accession number of the recombinant horse albumin is NP_001075972.1, the nucleotide sequence encoding the amino acid sequence of the recombinant horse albumin is as shown in SEQ ID NO.22, or has at least 98% identity to the nucleotide sequence as shown in SEQ ID NO.22.

Further, the amino acid sequence encoded by the Cas9 gene is as shown in SEQ ID NO.29, and the nucleotide sequence of the Cas9 gene is a nucleotide sequence encoding the amino acid sequence as shown in SEQ ID NO.29, including nucleotide sequences that have been publicly available or sequences optimized according to actual needs. Optionally, the nucleotide sequence is as shown in SEQ ID NO.27 or SEQ ID NO.28. In an embodiment of the present invention, the recombinant expression cassette of Cas9 and gRNA is located on the same nucleic acid construct.

The second aspect of the present invention provides use of the recombinant engineering bacterium in preparing a recombinant albumin and/or improving the expression level of the recombinant albumin.

The third aspect of the present invention provides a method for preparing a recombinant human albumin, the method comprising: preparing the recombinant albumin by fermentation using the recombinant engineering bacterium.

The present invention has the beneficial effects:
In the present invention, a recombinant engineering bacterium for preparing a recombinant human albumin is constructed, which significantly improves the expression level of the recombinant human albumin. Different from the traditional methods that directly improve a copy number of a recombinant protein coding gene and the like, the recombinant engineering bacterium of the present invention improves the structure of the cell wall to cause the structural defect of the cell wall, or ameliorates the transcription factor in the process of transcription to enhance the transcription level of the target gene. The recombinant engineering bacterium of the present invention significantly enhances a heterologous protein secretion ability of a recombinant engineering strain. The recombinant engineering bacterium exhibits a powerful potential in the aspect of improving the yield of a protein with a high economic value, and has wide application prospects.

### BRIEF DESCRIPTION OF THE DRAWINGS

The drawings described here are used for further understanding the present invention and constitute a part of the present invention. Exemplary embodiments of the present invention and their illustrations are used for explaining the present invention, but do not form improper limitation to the present invention. In the drawings,
Fig. 1 is a structural diagram of a recombinant expression cassette pPICZA-rHA encoding a recombinant albumin;
Fig. 2 is a structural diagram of a recombinant expression cassette pART202 encoding a Cas9 recombinant nucleic acid molecule and an SCW10-gRNA recombinant nucleic acid molecule;
Fig. 3 is a structural diagram of a recombinant expression cassette pART204 encoding a Cas9 recombinant nucleic acid molecule and a UTH1-gRNA recombinant nucleic acid molecule;
Fig. 4 is a structural diagram of a recombinant expression cassette pART208 encoding a BGL2 recombinant nucleic acid molecule and an RCE3 recombinant nucleic acid molecule;
Fig. 5 is a structural diagram of a recombinant expression cassette pART111 encoding an SUS1 recombinant nucleic acid molecule and an SUA7 recombinant nucleic acid molecule;
Fig. 6 is a structural diagram of a recombinant expression cassette pART222 encoding a Cas9 recombinant nucleic acid molecule and a YHP1-gRNA recombinant nucleic acid molecule;
Fig. 7 is a structural diagram of a recombinant expression cassette pART333 encoding a Cas9 recombinant nucleic acid molecule and an HDA1-gRNA recombinant nucleic acid molecule;
Fig. 8 is a diagram showing a standard curve result of an HIS4 gene in examples of the present invention;
Fig. 9 is a diagram showing a standard curve result of an rHA gene in examples of the present invention;
Fig. 10 is a diagram showing a gel electrophoresis result for expressing a recombinant human albumin by each recombinant engineering bacterium in operation A1 in examples;
Fig. 11 is a diagram showing a result for expressing a recombinant human albumin by each recombinant engineering bacterium in operation A1 in examples;
Fig. 12 is a diagram showing a result for expressing a recombinant human albumin by each recombinant engineering bacterium in operation A2 in examples;
Fig. 13 is a diagram showing a result for expressing a recombinant cat albumin by each recombinant engineering bacterium in operation A2 in examples;
Fig. 14 is a diagram showing a result for expressing a recombinant canine albumin by each recombinant engineering bacterium in operation A2 in examples;
Fig. 15 is a diagram showing a result for expressing a recombinant bovine albumin by each recombinant engineering bacterium in operation A2 in examples; and
Fig. 16 is a diagram showing a result for expressing a recombinant horse albumin by each recombinant engineering bacterium in operation A2 in examples.

### DETAILED DESCRIPTION OF THE EMBODIMENTS

The expression cassette refers to a gene expression system comprising all necessary elements required by exogenous protein expression, including promoters, exogenous gene cloning sites, signal peptide sequences, mature peptide coding sequences of target proteins, terminators, screening markers, etc.

The vector refers to autonomous DNA capable of introducing exogenous DNA into a host cell for replication or finally making exogenous gene DNA expressed. The vector mainly comprises a cloning vector and an expression vector, wherein the former is mainly used for replication, amplification and the like of genes, and the latter is mainly used for expression of a target gene.

The host cell refers to a cell receiving exogenous genes during the transformation or transduction.

The recombinant engineering bacterium refers to a fungi cell strainline for efficient expression of exogenous genes by using a genetic engineering method.

To more clearly illustrate the overall concept of the present invention, the present application will be described in detail in a mode of embodiments in conjunction with drawings below. The descriptions hereinafter give a large amount of specific details so as to provide the more thorough understanding of the present invention. However, it is obvious for those skilled in the art that the present invention will be implemented without one or more of these details. In other examples, to avoid the confusion with the present invention, some well-known technical features in the art are not described.

If specific conditions are not specified in the embodiments, the present invention is implemented in accordance with conventional conditions or conditions recommended by a manufacturer.

Unless otherwise specified, in the following embodiments, reagents or instruments used that do not indicate manufacturers are all conventional products that are commercially available.

Molecular biology experimental methods that are not specifically described in the following examples are all carried out according to kit and product instructions, or by reference to methods described in a book entitled "Molecular Cloning Experiment Guidelines" (Third Edition) written by J. Sambrook.

The sources of experimental materials and reagents involved in the present invention are as follows:
Strains: Pichia pastoris CBS7435 is purchased from American Type Culture Collection (ATCC) by Ningbo Mingzhou Biotechnology Co., Ltd., which is also called Komagataella phaffii NRRL Y-11430, with an accession number of 76273^{™}.

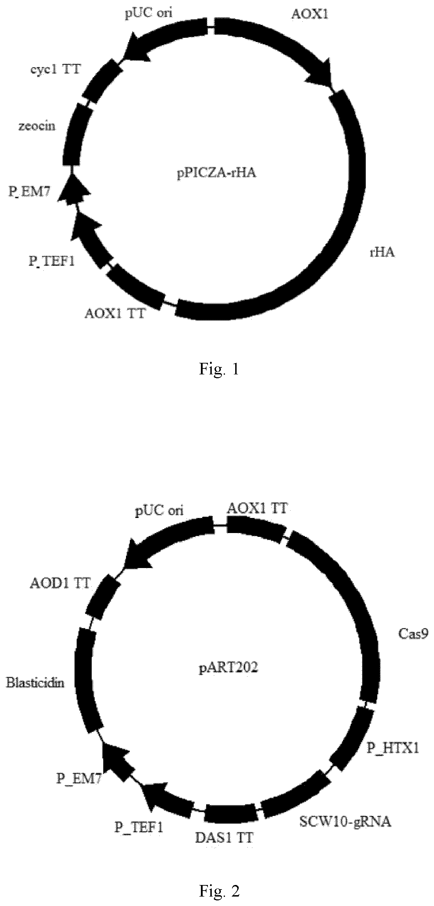

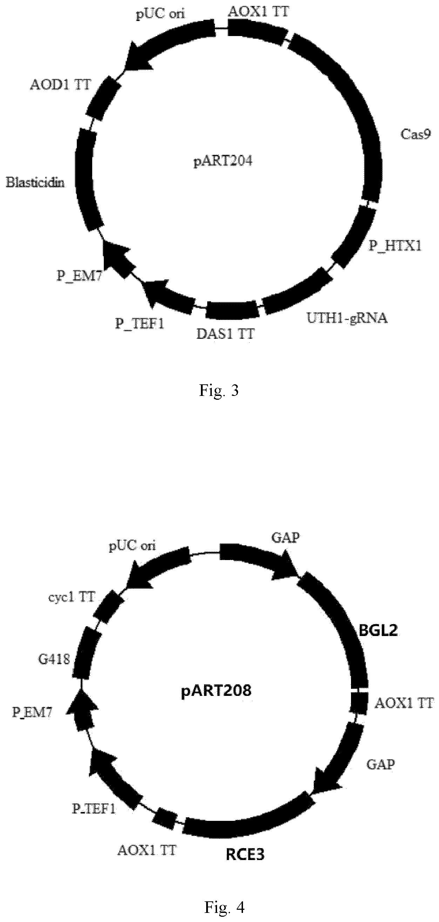

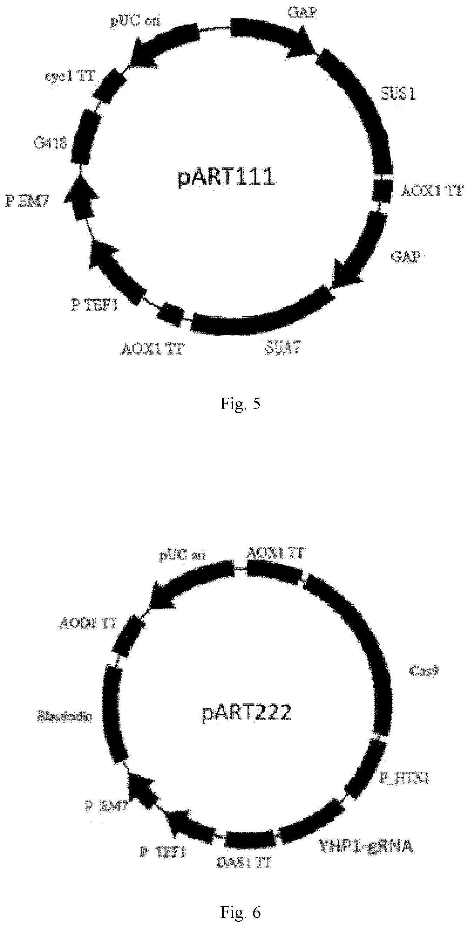

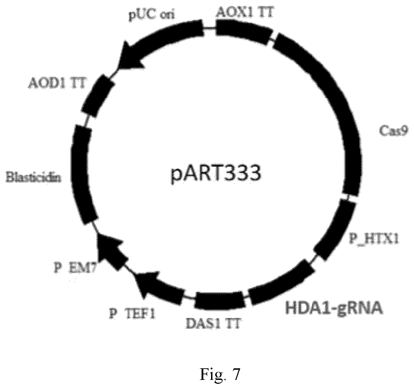

Expression vector: expression vectors pPICZA and pGAPZA are commercially available; the following expression cassettes are constructed in a lab: 1) an expression cassette encoding a recombinant nucleic acid molecule of at least one recombinant human albumin, wherein the structure of the expression cassette is as shown in Fig. 1; 2) an expression cassette encoding a Cas9 recombinant nucleic acid molecule and an SCW10-gRNA recombinant nucleic acid molecule, wherein the structure of the expression cassette is as shown in Fig. 2; 3) an expression cassette encoding a Cas9 recombinant nucleic acid molecule and a UTH1-gRNA recombinant nucleic acid molecule, wherein the structure of the expression cassette is shown in Fig. 3; 4) an expression cassette encoding a BGL2 recombinant nucleic acid molecule and an RCE3 recombinant nucleic acid molecule, wherein the structure of the expression cassette is as shown in Fig. 4; 5) an expression cassette encoding an SUS1 recombinant nucleic acid molecule and an SUA7 recombinant nucleic acid molecule, wherein the structure of the expression cassette is as shown in Fig. 5; 6) an expression cassette encoding a Cas9 recombinant nucleic acid molecule and a YHP1-gRNA recombinant nucleic acid molecule, wherein the structure of the expression cassette is as shown in Fig. 6; and 7) an expression cassette encoding a Cas9 recombinant nucleic acid molecule and an HDA1-gRNA recombinant nucleic acid molecule, wherein the structure of the expression cassette is as shown in Fig. 7.

### Enzyme and kit

Restriction endonuclease Bg1II is purchased from NEB; a plasmid extraction kit, an agarose gel extraction kit and a DNA product purification kit are purchased from Tiangen Biotechnology Co., Ltd.; a PrimeSTAR high fidelity DNA polymerase is purchased from Takara; Zeocin, Geneticin and Blasticidin are purchased from Thermo Fisher Scientific.

### Culture medium

Escherichia coli culture medium LB (1% (w/v) tryptone, 0.5% (w/v) yeast extract, 1% (w/v) NaCl, and pH 7.0).

Yeast culture medium YPD (1% (w/v) yeast extract, 2% (w/v) peptone, and 2% (w/v) glucose); yeast screening culture medium YPDZ (YPD + different concentrations of zeocin); YPDG (YPD + different concentrations of Geneticin); YPDB (YPD + different concentrations of Blasticidin).

Yeast culture medium BMGY (1% (w/v) yeast extract, 2% (w/v) peptone, 1.34% (w/v) YNB, 0.00004% (w/v) Biotin, and 1% glycerinum (V/V)).

Yeast induced culture medium BMMY (1% (w/v) yeast extract, 2% (w/v) peptone, 1.34% (w/v) YNB, 0.00004% (w/v) Biotin, and 0.4% methanol (V/V)); note: YNB is yeast nitrogen base; Biotin is a biotin.

Specific examples of operation A1:

### Example 1: Construction of recombinant human albumin Pichia pastoris CBS7435 strain

A biology company was entrusted to directly synthesize a nucleic acid molecule (SEQ ID NO.30) encoding a saccharomyces cerevisiae a mating factor signal peptide and a nucleic acid molecule (a nucleotide sequence was as shown in SEQ ID NO.17) encoding a recombinant human albumin, and then the synthesized nucleic acid molecules were linked into a plasmid pPICZA digested by EcoRI and NotI to form an expression vector named pPICZA-rHA (as shown in Fig. 1).

The expression vector was linearized by using DNA restriction endonuclease BglII, Pichia pastoris CBS7435 was transformed by electric shock, cells were coated onto a selective plate YPDZ containing different high concentrations of zeocin antibiotics and then cultured at 30°C until colonies appeared. By colony polymerase chain reaction (PCR), positive transformants were identified and then stored in a glycerinum tube.

The genomic DNA of 4 randomly selected transformants was extracted with a yeast genomic extraction kit, a real-time fluorescent quantitative PCR was carried out by respectively utilizing vectors containing endogenous gene HIS4 and exogenous gene recombinant human albumins present in a form of single copy by using a double standard curve method, a standard curve was established, and the copy number of the recombinant human albumin in each transformant was detected.

A double standard curve was established by respectively using a pPIC9K vector (purchased from Invitrogen) containing a single copy of HIS4 gene and a pPICZA-rHA vector containing a single copy of recombinant human albumin gene as standards, the copy number of a starting plasmid was 10⁹, 10-fold dilution was carried out, with 6 dilution gradients in total, and 2 parallel samples for each gradient were used as an amplified standard curve. Each copy of extracted transformant genome was diluted to 10 ng/µL as a PCR template.

The fluorescent quantitative PCR system is seen in Table 1, and specific nucleotide sequences of upstream primers and downstream primers are seen in Table 2.

**Table 1**

| | |
|---|---|
| FastSYBR Mixture | 10 µL |
| Upstream primer | 0.4 µL |
| Downstream primer | 0.4 µL |
| DNA template | 5 µL |
| RNase-Free ddH₂O | 4.2 µL |

**Table 2**

| | |
|---|---|
| HIS4 upstream primer | GGTTGAACAAACAGGTGTTG |
| HIS4 downstream primer | GTTCCTTGGTGTATCCTGGC |
| rHA upstream primer | GTACGAAATCGCCAGACG |
| rHA downstream primer | CCTTTCGCCGAACTTCTG |

Fluorescence quantitative PCR reaction parameters:
Stage 1: 95°C for 3 min; stage 2: 95°C for 5 s, 60°C for 20 s, repeat for 40 cycles; stage 3: 95°C for 10 s, 72°C for 5 min, and 97°C for 1 s.

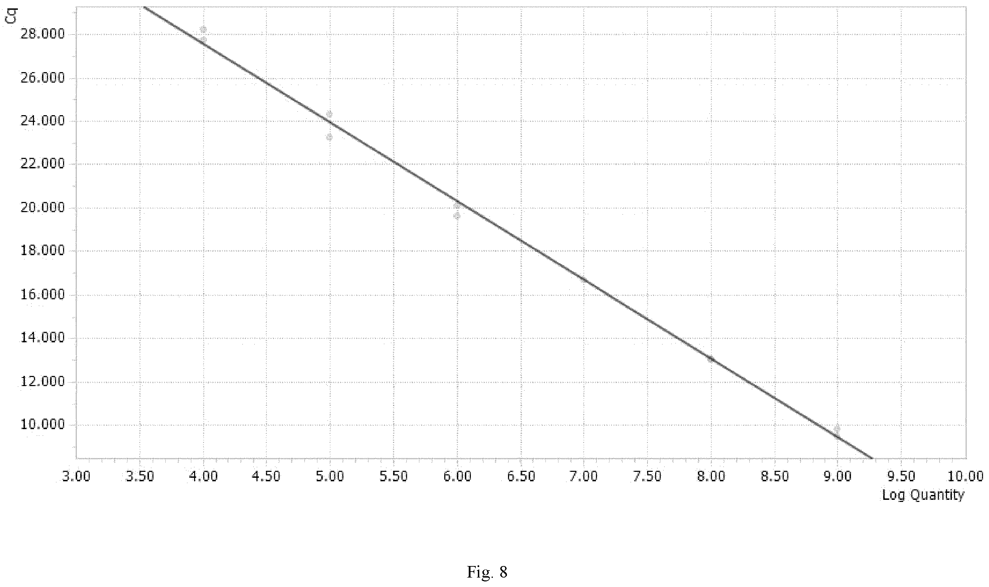

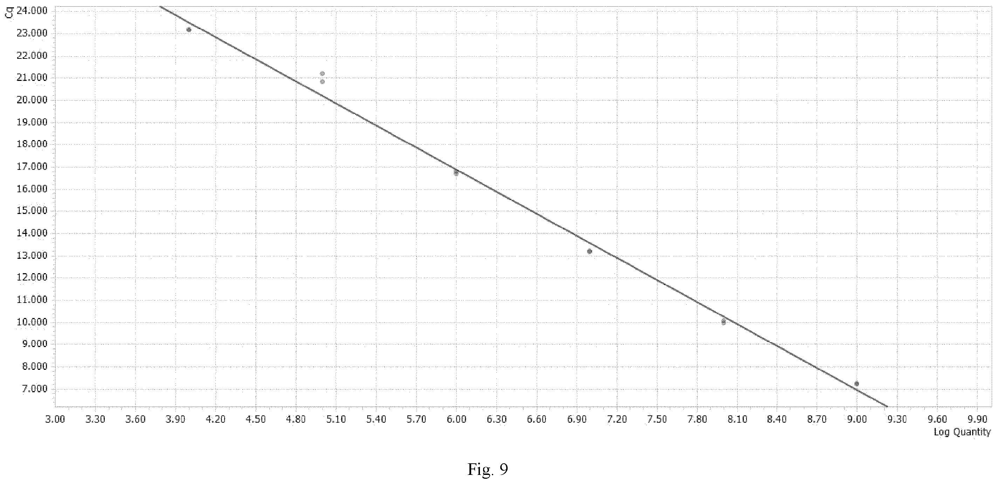

The cycle number Ct of each copy of transformant test solution was respectively substituted into a standard curve (an HIS4 gene standard curve was as shown in Fig. 8, and an rHA gene standard curve was as shown in Fig. 9) to obtain copy number concentrations of starting templates of endogenous gene HIS4 and exogenous gene rHA in a genome. Through a ratio of the copy number of the starting template of the exogenous gene to the copy number of the starting template of the endogenous gene, the copy numbers of 4 transformants obtained by screening after transformation were calculated to be 1 copy, 3 copies, 3 copies and 2 copies, respectively.

### Example 2: Construction of Cas9 and gRNA co-expression vector

In this example, to establish a proper genome editing CRISPR system, it was attempted to construct a Cas9 and gRNA co-expression vector by using double promoters pHTX1 (a nucleotide acid sequence was as shown in SEQ ID NO.34) (Weninger A, Hatzl AM, Schmid C, Vogl T, Glieder A. Combinatorial optimization of CRISPR/Cas9 expression enables precision genome engineering in the methylotrophic yeast Pichia pastoris. J Biotechnol. 2016 Oct. 10; 235:139-49.).

A biological company was entrusted to directly synthesize a recombinant expression cassette encoding a Cas9 gene and an SCW10-gRNA gene, and the synthesized recombinant expression cassette was linked to a plasmid pPICZA digested by using BglII and BamHI to form an expression vector named pART201. A biological company was entrusted to replace a zeocin resistance gene in pART201 with a Blasticidin resistance gene (a nucleotide sequence was as shown in SEQ ID NO.33) by utilizing a gene homologous recombination technology to form an expression vector named pART202 (as shown in Fig. 2).

A biological company was entrusted to directly synthesize a recombinant expression cassette encoding a Cas9 gene and a UTH1-gRNA gene, and the synthesized recombinant expression cassette was linked to a plasmid pPICZA digested by using BglII and BamHI to form an expression vector named pART203. A biological company was entrusted to replace a zeocin resistance gene in pART203 with a Blasticidin resistance gene (a nucleotide sequence was as shown in SEQ ID NO.33) utilizing a gene homologous recombination technology to form an expression vector named pART204 (as shown in Fig. 3).

### Example 3: Construction of SCW10 and UTH1 gene targeted knockout strain

To prepare SCW10 knockout donor DNA, the following operations were performed: left and right homologous arm fragments of an SCW10 gene were obtained by PCR with Pichia pastoris CBS7435 genome DNA as a template and SCW10-UP-F/SCW10-UP-R and SCW10-DN-F/SCW10-DN-R as primers; and then SCW10 knockout donor DNA was obtained by utilizing an overlap extension PCR technology with SCW10-UP-F and SCW10-DN-R as primers and a mixture of the left and right homologous arm fragments as a DNA template.

To prepare UTH1 knockout donor DNA, the following operations were performed: left and right homologous arm fragments of a UTH1 gene were obtained by PCR with Pichia pastoris CBS7435 genome DNA as a template and UTH1-UP-F/UTH1-UP-R and UTH1-DN-F/UTH1-DN-R as primers; and then UTH1 knockout donor DNA was obtained by utilizing an overlap extension PCR technology with UTH1-UP-F and UTH1-DN-R as primers and a mixture of the left and right homologous arm fragments as a DNA template.

The PCR reaction system is seen in Table 3, and specific nucleotide sequences of upstream primers and downstream primers are seen in Table 4.

**Table 3**

| | |
|---|---|
| PrimeSTAR HS | 12.5 µl |
| Upstream primer | 1 µl |
| Downstream primer | 1 µl |
| DNA template | 5 µl |
| ddH₂O | 5.5 µl |

**Table 4**

| | |
|---|---|
| SCW10-UP-F | CTAAGAAATCAGATTCGAGCATCG |
| SCW10-UP-R | CGAAGATCAGGCTTCCATCGCGGTTGATAAAAGGAATGAC |
| SCW10-DN-F | GCGATGGAAGCCTGATCTTCGATTCTTCTC |
| SCW10-DN-R | CAGATGCTGATGGCCAAATCGGGAGGTATC |
| UTH1-UP-F | GAGTGTATGAATTAAATTAGGTACC |
| UTH1-UP-R | GGGTATAAGTGGGAGCGTATTGAGAAGCACGGTTAAAAAATG |
| UTH1-DN-F | ACGCTCCCACTTATACCCAATATACTGAG |
| UTH1-DN-R | ATACCCCTCGTATCTCCTACAACC |

2 µg of Cas9 and gRNA co-expression vector and 2 µg of donor DNA were respectively used to co-transform a recombinant engineering bacterium 2 in example 1, then the above transformed recombinant engineering bacterium 2 was coated onto a selective plate YPDB containing Blasticidin antibiotics and then cultured at 30°C until colonies appeared. By colony PCR, positive transformants were identified as recombinant engineering bacteria 5-6. Specific information is seen in Table 5.

### Example 4: Construction of BGL2 and RCE3 co-expression strain

A biological company was entrusted to directly synthesize a recombinant expression cassette encoding a BGL2 gene (a nucleotide sequence was as shown in SEQ ID NO.5, and an amino acid sequence was as shown in SEQ ID NO.6), and then the synthesized recombinant expression cassette was linked to a plasmid pGAPZA to form an expression vector named pART205. A biological company was entrusted to replace a zeocin resistance gene in pART205 with a geneticin G418 resistance gene (a nucleotide sequence was as shown in SEQ ID NO.32) to form an expression vector named pART206.

A biological company was entrusted to directly synthesize a recombinant expression cassette encoding a BGL2 gene (a nucleotide sequence was as shown in SEQ ID NO.5, and an amino acid sequence was as shown in SEQ ID NO.6) and an RCE3 gene (a nucleotide sequence was as shown in SEQ ID NO.7, and an amino acid sequence was as shown in SEQ ID NO.8), and then the synthesized recombinant expression cassette was linked to a plasmid pGAPZA to form an expression vector named pART207. A biological company was entrusted to replace a zeocin resistance gene in pART205 with a geneticin G418 resistance gene (a nucleotide sequence was as shown in SEQ ID NO.32) to form an expression vector named pART208 (as shown in Fig. 4).

Expression vectors pART206 and pART208 were respectively linearized using DNA restriction endonuclease BglII to respectively transform a recombinant engineering bacterium 6 through electric shock, and cells were coated onto a selective plate YPDG containing geneticin G418 antibiotics and then cultured at 30°C until colonies appeared. By colony PCR, positive transformants were identified as recombinant engineering bacteria 7-8. Specific information is seen in Table 5.

**Table 5**

| | Copy number of recombinant human albumin | Knock out SCW10 gene | Knock out UTH1 gene | Overexpress BGL2 gene | Overexpress RCE3 gene |
|---|---|---|---|---|---|
| Recombinant engineering bacterium 1 | 1 | No | No | No | No |
| Recombinant engineering bacterium 2 | 3 | No | No | No | No |
| Recombinant engineering bacterium 3 | 3 | No | No | No | No |
| Recombinant engineering bacterium 4 | 2 | No | No | No | No |
| Recombinant engineering bacterium 5 | 3 | Yes | No | No | No |
| Recombinant engineering bacterium 6 | 3 | Yes | Yes | No | No |
| Recombinant engineering bacterium 7 | 3 | Yes | Yes | Yes | No |
| Recombinant engineering bacterium 8 | 3 | Yes | Yes | Yes | Yes |

### Example 5: Expression level of recombinant human albumin in recombinant engineering bacteria

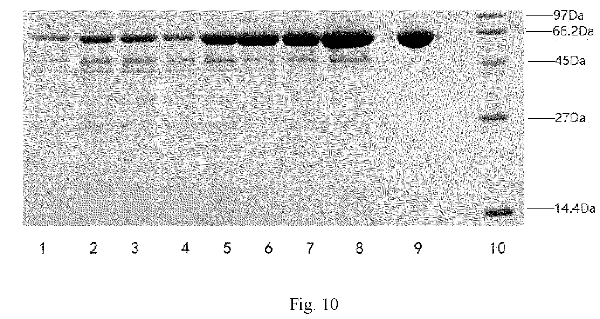

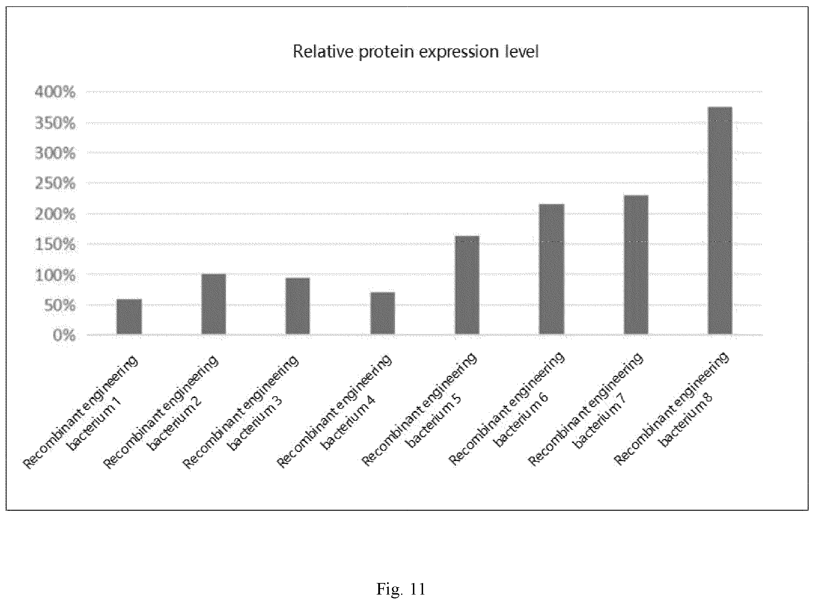

Recombinant engineering bacteria 1-8 were respectively taken and inoculated into 40 mL of BMGY culture solution to be cultured for overnight at 30°C under 220 rpm, a bacterial solution was taken and centrifuged, a precipitate was resuspended into 40 mL of BMMY culture solution and then started to be induced at 25°C under 220 rpm, 0.4% methanol was added every 24 h, the bacterial solution was centrifuged for 5 min under 10,000 rpm after 72 h, and then supernatant was collected. The supernatant was subjected to sodium dodecyl sulfate-polyacrylamide gel electrophoresis (SDS-PAGE) detection. The electrophoretogram is as shown in Fig. 10, and specific results are seen in Table 6 and as shown in Fig. 11.

The results showed that when SCW10 and UTH1 genes were knocked out, and BGL2 and RCE3 genes were simultaneously overexpressed, the expression level of the recombinant human albumin in the recombinant engineering bacterium 2 was used as control, with the expression levels of up to 374%.

**Table 6**

| Strains | Copy number of recombinant human albumin | Knock out SCW10 gene | Knock out UTH1 gene | Overexpress BGL2 gene | Overexpress RCE3 gene | Relative protein expression level |
|---|---|---|---|---|---|---|
| Recombinant engineering bacterium 1 | 1 | No | No | No | No | 59% |
| Recombinant engineering bacterium 2 | 3 | No | No | No | No | 100% |
| Recombinant engineering bacterium 3 | 3 | No | No | No | No | 95% |
| Recombinant engineering bacterium 4 | 2 | No | No | No | No | 71% |
| Recombinant engineering bacterium 5 | 3 | Yes | No | No | No | 163% |
| Recombinant engineering bacterium 6 | 3 | Yes | Yes | No | No | 216% |
| Recombinant engineering bacterium 7 | 3 | Yes | Yes | Yes | No | 229% |
| Recombinant engineering bacterium 8 | 3 | Yes | Yes | Yes | Yes | 374% |

### Operation A2 examples:

### Example 6: Construction of SUS1 and SUA7 co-expression strain

A BGL2 gene in example 4 was replaced with an SUS1 gene (a nucleotide sequence is as shown in SEQ ID NO.9, and an amino acid sequence is as shown in SEQ ID NO.10), an RCE3 gene in example 4 was replaced with an SUA7 gene (a nucleotide sequence is as shown in SEQ ID NO.11, and an amino acid sequence is as shown in SEQ ID NO.12), and experimental steps in example 4 were repeated to form an SUS1 and SUA7 co-expression vector named pART111 (Fig. 5). By colony PCR, the obtained positive transformant was identified as a recombinant engineering bacterium 9.

### Example 7: Construction of Cas9 and gRNA co-expression vector (YHP1 and HDA1)

An SCW10 gene in example 2 was replaced with a YHP1 gene, and the steps in example 2 were repeated to obtain an expression vector named pART222 (Fig. 6); a UTH1 gene in example 2 was replaced with an HDA1 gene, and the steps in example 2 were repeated to obtain an expression vector named pART333 (Fig. 7), wherein, the YHP1-gRNA gene sequence was as shown in SEQ ID NO.25; the HDA1-gRNA gene sequence was as shown in SEQ ID NO.26, and the nucleotide sequence of Cas9 was as shown in SEQ ID NO.28.

### Example 8: Construction of YHP1 and HDA1 gene targeted knockout strain

An SCW10 gene in example 3 was replaced with a YHP1 gene, and the steps in example 3 were repeated to obtain an expression vector pART222; a UTH1 gene in example 3 was replaced with an HDA1 gene, and the steps in example 3 were repeated to obtain an expression vector pART333. Specific nucleotide sequences of upstream primers and downstream primers are seen in Table 7.

**Table 7**

| | |
|---|---|
| YHP1-UP-F | TGACGATACTGAAGTATTGTCG |
| YHP1-UP-R | TTTGCACACCGCAAAGTATTAACCAAAGG |
| YHP1-DN-F | GGTGTGCAAATTACTATCTTAG |
| YHP1-DN-R | GGACTATCACACTCTTGCC |
| HDA1-UP-F | TTTCCAACGTTTGTGAAG |
| HDA1-UP-R | TCCCTTTGTCGAAGGACACAGAGGAGGA |
| HDA1-DN-F | GACAAAGGGATTAACAGTAG |
| HDA1-DN-R | CTGGAAGATGAACTGAATAC |

2 µg of Cas9 and YHP1-gRNA co-expression vector and 2 µg of YHP1 knockout donor DNA were used to co-transform the recombinant engineering bacterium 9 in example 6, and then the transformed recombinant engineering bacterium was coated onto a selective plate YPDB containing Blasticidin antibiotics and cultured at 30°C until colonies appeared. By colony PCR, the positive transformant was identified as a recombinant engineering bacterium 10.

2 µg of Cas9 and HDA1-gRNA co-expression vector and 2 µg of HDA1 knockout donor DNA were used to co-transform the recombinant engineering bacterium 9 in example 6, and then the transformed recombinant engineering bacterium was coated onto a selective plate YPDB containing Blasticidin antibiotics and cultured at 30°C until colonies appeared. By colony PCR, the positive transformant was identified as a recombinant engineering bacterium 11.

2 µg of Cas9 and HDA1-gRNA co-expression vector and 2 µg of HDA1 knockout donor DNA were used to co-transform the recombinant engineering bacterium 10 in example 3, and then the transformed recombinant engineering bacterium was coated onto a selective plate YPDB containing Blasticidin antibiotics and cultured at 30°C until colonies appeared. By colony PCR, the positive transformant was identified as a YHP1 and HDA1 knockout recombinant engineering bacterium 12.

### Example 9: Construction of recombinant albumin Pichia pastoris strain

A biology company was entrusted to directly synthesize a nucleic acid molecule (SEQ ID NO.31) encoding a saccharomyces cerevisiae a mating factor signal peptide, a nucleic acid molecule (a nucleotide sequence as shown in SEQ ID NO.18) encoding a recombinant human albumin, a nucleic acid molecule (a nucleotide sequence as shown in SEQ ID NO.19) encoding a recombinant cat albumin, a nucleic acid molecule (a nucleotide sequence as shown in SEQ ID NO.20) encoding a recombinant canine albumin, a nucleic acid molecule (a nucleotide sequence as shown in SEQ ID NO.21) encoding a recombinant bovine albumin and a nucleic acid molecule (a nucleotide sequence as shown in SEQ ID NO.22) encoding a recombinant horse albumin, and then the synthesized nucleic acid molecules were linked to the plasmid pPICZA digested by EcoRI and NotI to form expression vectors named pPICZA-I to pPICZA-V in sequence.

The expression vectors were linearized by DNA restriction endonuclease BglII, Pichia pastoris CBS7435 and recombinant engineering bacteria 9-12 were transformed by electric shock, cells were coated onto a selective plate YPDZ containing different high concentrations of zeocin antibiotics and then cultured at 30°C until colonies appeared. By colony PCR, positive transformants were identified and then stored in a glycerinum tube to obtain recombinant engineering bacteria 13-37. The specific information of the recombinant engineering bacteria is seen in Table 8.

### Example 10: Expression levels of recombinant albumins in recombinant engineering bacteria

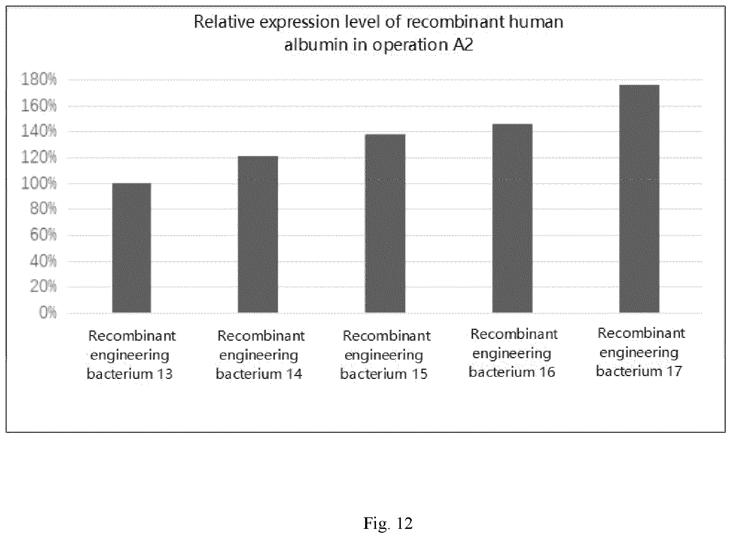

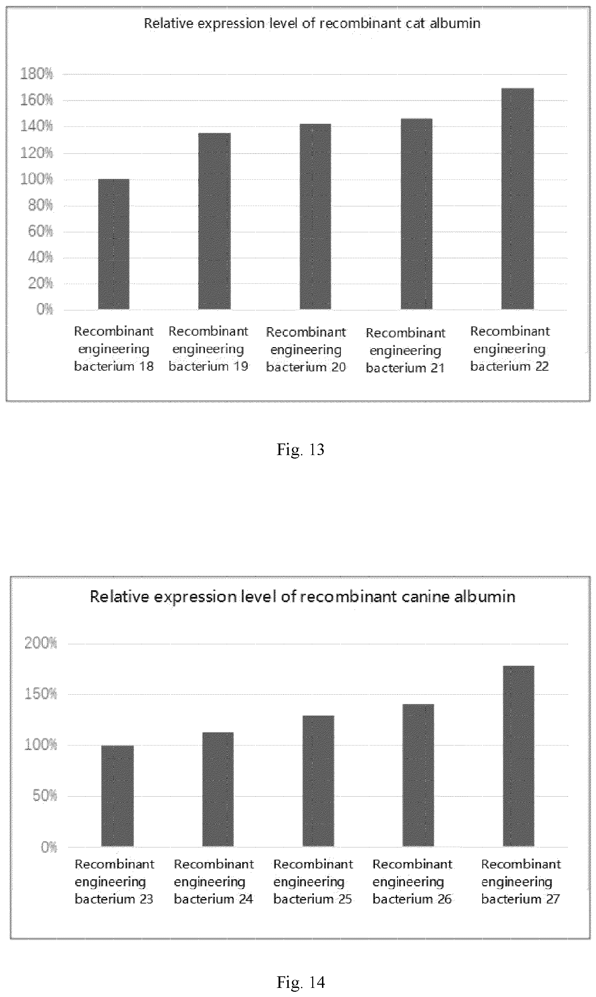

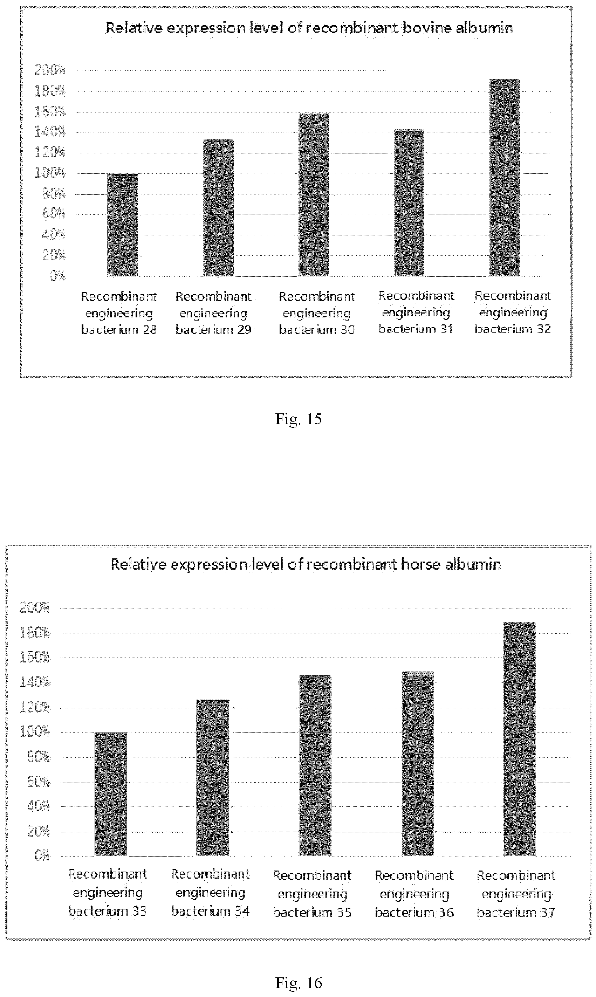

The recombinant engineering bacteria 1-8 in example 5 were replaced with recombinant engineering bacteria 13-37, and experimental steps in example 5 were repeated. The results are seen in Table 8 and as shown in Fig. 12-Fig. 16, wherein, the relative expression levels of the recombinant human albumin, the recombinant cat albumin, the recombinant canine albumin, the recombinant bovine albumin and the recombinant horse albumin respectively used recombinant engineering bacteria 13, 18, 23, 28 and 33 as controls.

The results show that when SUS1 and SUA7 genes were overexpressed, and YHP1 and HDA1 genes were simultaneously knocked out, the relative expression level of the recombinant albumin of each recombinant engineering bacterium is the highest.

**Table 8**

| Strains | Type of recombinant albumin | Overexpress SUS1 gene | Overexpress SUA7 gene | Knock out YHP1 gene | Knock out HDA1 gene | Relative expression level of recombinant albumin |
|---|---|---|---|---|---|---|
| Recombinant engineering bacterium 13 | Recombinant human albumin | No | No | No | No | 100% |
| Recombinant engineering bacterium 14 | Recombinant human albumin | Yes | Yes | No | No | 121% |
| Recombinant engineering bacterium 15 | Recombinant human albumin | Yes | Yes | Yes | No | 138% |
| Recombinant engineering bacterium 16 | Recombinant human albumin | Yes | Yes | No | Yes | 146% |
| Recombinant engineering bacterium 17 | Recombinant human albumin | Yes | Yes | Yes | Yes | 176% |
| Recombinant engineering bacterium 18 | Recombinant cat albumin | No | No | No | No | 100% |
| Recombinant engineering bacterium 19 | Recombinant cat albumin | Yes | Yes | No | No | 135% |
| Recombinant engineering bacterium 20 | Recombinant cat albumin | Yes | Yes | Yes | No | 142% |
| Recombinant engineering bacterium 21 | Recombinant cat albumin | Yes | Yes | No | Yes | 146% |
| Recombinant engineering bacterium 22 | Recombinant cat albumin | Yes | Yes | Yes | Yes | 169% |
| Recombinant engineering bacterium 23 | Recombinant canine albumin | No | No | No | No | 100% |
| Recombinant engineering bacterium 24 | Recombinant canine albumin | Yes | Yes | No | No | 113% |
| Recombinant engineering bacterium 25 | Recombinant canine albumin | Yes | Yes | Yes | No | 129% |
| Recombinant engineering bacterium 26 | Recombinant canine albumin | Yes | Yes | No | Yes | 141% |
| Recombinant engineering bacterium 27 | Recombinant canine albumin | Yes | Yes | Yes | Yes | 178% |
| Recombinant engineering bacterium 28 | Recombinant bovine albumin | No | No | No | No | 100% |
| Recombinant engineering bacterium 29 | Recombinant bovine albumin | Yes | Yes | No | No | 133% |
| Recombinant engineering bacterium 30 | Recombinant bovine albumin | Yes | Yes | Yes | No | 158% |
| Recombinant engineering bacterium 31 | Recombinant bovine albumin | Yes | Yes | No | Yes | 143% |
| Recombinant engineering bacterium 32 | Recombinant bovine albumin | Yes | Yes | Yes | Yes | 192% |
| Recombinant engineering bacterium 33 | Recombinant horse albumin | No | No | No | No | 100% |
| Recombinant engineering bacterium 34 | Recombinant horse albumin | Yes | Yes | No | No | 126% |
| Recombinant engineering bacterium 35 | Recombinant horse albumin | Yes | Yes | Yes | No | 146% |
| Recombinant engineering bacterium 36 | Recombinant horse albumin | Yes | Yes | No | Yes | 149% |
| Recombinant engineering bacterium 37 | Recombinant horse albumin | Yes | Yes | Yes | Yes | 189% |

The above descriptions are only embodiments of the present invention, but not intended to limit the present invention. For those skilled in the art, various changes and variations are made to the present invention. Any modifications, equivalent replacements, improvements and the like made within the spirit and principle of the present invention should be included within the scope of the claims of the present invention.

## Claims

1. A recombinant engineering bacterium for preparing a recombinant albumin, wherein the recombinant engineering bacterium is obtained by any one of the following operations while expressing the recombinant albumin:
A1, conducting targeted knockout on one or more genes of SCW10 and UTH1 by utilizing a CRISPR-Cas9 gene editing technology, and overexpressing and integrating one or more genes of BGL2 and RCE3 into a strain, wherein the recombinant engineering bacterium at least comprises 1 copy of nucleotide sequence encoding the recombinant albumin, and selecting Pichia pastoris as the recombinant engineering bacterium;
A2, simultaneously overexpressing and integrating an SUS1 gene and an SUA7 gene into a strain, and conducting targeted knockout on one or more genes of YHP1 and HDA1 by utilizing the CRISPR-Cas9 gene editing technology, wherein the recombinant albumin is at least one selected from a recombinant human albumin, a recombinant cat albumin, a recombinant canine albumin, a recombinant bovine albumin and a recombinant horse albumin, wherein the amino acid sequence encoded by the SUS1 gene is as shown in SEQ ID NO.10; the amino acid sequence encoded by the SUA7 gene is as shown in SEQ ID NO.12; the amino acid sequence encoded by the YHP1 gene is as shown in SEQ ID NO.14; the amino acid sequence encoded by the HDA1 gene is as shown in SEQ ID NO. 16 , and Pichia pastoris is selected as the recombinant engineering bacterium.

2. The recombinant engineering bacterium according to claim 1, wherein the amino acid sequence encoded by each gene in the operation A1 is as follows:
the amino acid sequence encoded by the SCW10 gene is as shown in SEQ ID NO.2;
the amino acid sequence encoded by the UTH1 gene is as shown in SEQ ID NO.4;
the amino acid sequence encoded by the BGL2 gene is as shown in SEQ ID NO.6;
the amino acid sequence encoded by the RCE3 gene is as shown in SEQ ID NO.8.

3. The recombinant engineering bacterium according to claim 1, wherein the nucleotide sequence of each gene is as follows:
the nucleotide sequence of the SCW10 gene is as shown in SEQ ID NO.1;
the nucleotide sequence of the UTH1 gene is as shown in SEQ ID NO.3;
the nucleotide sequence of the BGL2 gene is as shown in SEQ ID NO.5;
the nucleotide sequence of the RCE3 gene is as shown in SEQ ID NO.7;
the nucleotide sequence of the SUS1 gene is as shown in SEQ ID NO.9;
the nucleotide sequence of the SUA7 gene is as shown in SEQ ID NO.11;
the nucleotide sequence of the YHP1 gene is as shown in SEQ ID NO.13; and
the nucleotide sequence of the HDA1 gene is as shown in SEQ ID NO.15.

4. The recombinant engineering bacterium according to claim 1, wherein in the operation A1 the recombinant engineering bacterium also comprises a drug resistance gene fragment and/or a signal peptide sequence.

5. The recombinant engineering bacterium according to claim 1, wherein the conducting targeted knockout of relevant genes by utilizing the CRISPR-Cas9 gene editing technology, comprises the following steps:
S1: constructing a Cas9 and gRNA co-expression plasmid;
S2: preparing donor DNA;
S3: preparing a competent cell; and
S4: constructing any one of the following strains:
S4-1: knocking out one or more genes of SCW10 and UTH1; and
S4-2: knocking out one or more genes of YHP1 and HDA1.

6. The recombinant engineering bacterium according to claim 1, wherein a recombinant expression cassette encoding one or more genes of BGL2 and RCE3 is located on a nucleic acid construct with the same selective marker or a nucleic acid construct with different selective markers; and a recombinant expression cassette encoding one or more genes of SUS1 and SUA7 is located on a nucleic acid construct with the same selective marker or a nucleic acid construct with different selective markers.

7. The recombinant engineering bacterium according to claim 6, wherein the nucleic acid construct comprises any one selected from pHIL-D2, pPIC3.5, pHIL-S1, pPIC9, pPink-LC, pPink-HC, pGAPZA and pGAPZaA plasmids.

8. The recombinant engineering bacterium according to claim 1, wherein the recombinant engineering bacterium is Pichia pastoris CBS7435.

9. Use of the recombinant engineering bacterium according to any one of claims 1-8 for preparing a recombinant albumin and/or improving the expression level of the recombinant albumin.

10. A method for preparing a recombinant albumin, wherein the method includes: using the recombinant engineering bacterium according to any one of claims 1-8 for fermentation, so as prepare the recombinant albumin.
